# EUROPEAN PATENT APPLICATION

(11) **EP 4 557 309 A1**
(43) Date of publication of application: **21.05.2025**
(21) Application number: 23857781.1
(22) Date of filing: 25.08.2023
(51) Int. Cl.: G16H 50/20, G16H 50/30, G16H 30/20, G16H 80/00, G16H 40/60, A61B 5/346, A61B 5/00

(54) **METHOD AND APPARATUS WHICH PROVIDE USER INTERFACE FOR ELECTROCARDIOGRAM ANALYSIS**

(30) Priority: 25.08.2022 KR 20220106668; 24.08.2023 KR 20230111232
(71) Applicant: Medical AI Co., Ltd., Seoul 06180 (KR)
(72) Inventor: KWON, Joonmyoung, Seoul 06180 (KR)
(74) Representative: Patentanwaltskanzlei Matschnig & Forsthuber OG
(86) International application number: PCT/KR2023/012642
(87) International publication number: WO 2024/043749

(57) **Abstract**

According to one embodiment of the present disclosure, there are disclosed a method, program and apparatus for providing a user interface for electrocardiogram analysis. The method may comprise: obtaining bio-data of an electrocardiogram reading target; and displaying a first control graphic configured to switch a visual representation and state according to the result of the electrocardiogram analysis performed based on the obtained bio-data in the first area of the user interface. Furthermore, the first control graphic may be constructed for each type of disease or electrocardiogram feature that can be read from an electrocardiogram.

## Description

### Technical Field

The content of the present disclosure relates to user interface technology, and more particularly, to a user interface that provides a convenient function for analyzing an electrocardiogram in an electrocardiogram reading service.

### Background Art

An electrocardiogram test is a test that records the electrical activities of the heart. An electrocardiogram test is a relatively simple and cost-effective test method, and can check the health of the heart, which plays an important role in the early diagnosis and management of heart disease. For example, the electrocardiogram signal measured via an electrocardiogram test may be used to check whether each part of the heart is functioning normally, whether the size and location of the heart are normal, and whether there is damage to the heart muscle. Furthermore, based on this checking, the electrocardiogram signal may be used to diagnose various heart-related problems and predict a person's health status. This process is called electrocardiogram reading or analysis.

Meanwhile, with the development of artificial intelligence technology, attempts to read electrocardiograms using artificial intelligence technology are increasing. One of the representative examples is a service that predicts various heart diseases by inputting an electrocardiogram to a neural network-based model. However, it is still difficult for artificial intelligence technology to completely replace humans, and the medical field cannot help but be more cautious about the application thereof. Therefore, it is necessary to provide a computing environment in which artificial intelligence-based reading is performed and also a human professional can smoothly read electrocardiograms.

### Disclosure

### Technical Problem

An object of the present disclosure is to provide a method of providing a user interface that allows a professional to easily perform electrocardiogram reading with simple operations and to intuitively become aware of information and review reading results.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method of providing a user interface for electrocardiogram analysis. The method may comprise: obtaining bio-data of an electrocardiogram reading target; and displaying a first control graphic configured to switch a visual representation and state according to the result of the electrocardiogram analysis performed based on the obtained bio-data in the first area of the user interface. Furthermore, the first control graphic may be constructed for each type of disease or electrocardiogram feature that can be read from an electrocardiogram.

Alternatively, the first control graphic may be a button-type graphic configured to dynamically switch a visual representation and state in response to a command to input the result of the electrocardiogram analysis. Furthermore, the command may be generated automatically when an operation of performing the electrocardiogram analysis is completed, or may be generated through a user operation for inputting the result of the electrocardiogram analysis.

Alternatively, the first control graphic may include at least one of: a first sub-control graphic configured to dynamically switch a visual representation and state according to an analysis result obtained by inputting electrocardiogram data, included in the obtained bio-data, to a pre-trained artificial intelligence model; a second sub-control graphic configured to dynamically switch a visual representation and state according to an analysis result obtained according to a logic predetermined for each type of disease or electrocardiogram feature that can be read from the electrocardiogram; and a third sub-control graphic configured to dynamically switch a visual representation and state based on a user operation for inputting an analysis result obtained by a user who analyzes the electrocardiogram.

Alternatively, when the analysis result in which a disease that can be read from an electrocardiogram has occurred is obtained via the artificial intelligence model, the first sub-control graphic may be switched from an OFF state to an ON state. Furthermore, when the analysis result in which a disease that can be read from an electrocardiogram has not occurred is obtained via the artificial intelligence model, the first sub-control graphic may be maintained in an OFF state.

Alternatively, when being switched from an OFF state to an ON state, the first sub-control graphic may dynamically switch a color according to the severity that can be read based on the electrocardiogram analysis result obtained by the artificial intelligence model.

Alternatively, when a user input for a selection method is received, the first sub-control graphic may be switched to an ON state or an OFF state.

Alternatively, the predetermined logic may derive a feature associated with at least one of the rhythm and shape of an electrocardiogram signal included in the obtained bio-data, and may determine whether a condition preset to determine the onset of a specific disease is satisfied by analyzing the derived feature.

Alternatively, when it is determined that the feature satisfies the condition, the second sub-control graphic may be switched from an OFF state to an ON state. When it is determined that the feature does not satisfy the condition, the second sub-control graphic may be maintained in an OFF state.

Alternatively, when being switched from an OFF state to an ON state, the second sub-control graphic may dynamically switch a color according to the severity that can be read based on the analysis result obtained according to the predetermined logic.

Alternatively, when a user input for a selection method is received, the second sub-control graphic may be switched to an ON state or an OFF state.

Alternatively, when a user input for a selection method is received, the third sub-control graphic may dynamically switch at least one of a color and a state.

Alternatively, the method may further comprise displaying a second control graphic configured to display a reading text generated by synthesizing analysis results displayed via the first control graphic in the second area of the user interface.

Alternatively, the reading text may include tags assigned to analysis results, respectively, in order to distinguish reading results corresponding to the analysis results displayed via the first sub-control graphic in an ON state, the analysis results displayed via the second sub-control graphic in an ON state, and the analysis results displayed via the third sub-control graphic in an ON state.

Alternatively, the method may further comprise displaying a third control graphic configured to represent reading results, generated by synthesizing the analysis results displayed via the first control graphic, in color in the third area of the user interface.

Alternatively, the third control graphic may dynamically switch a color according to the severity read by synthesizing the analysis results displayed via the first control graphic.

Alternatively, when the number of leads of an electrocardiogram signal included in the bio-data is determined, the first control graphic may be reconstructed for each type of disease or electrocardiogram feature that can be read based on the number of leads of the electrocardiogram signal.

According to one embodiment of the present disclosure for achieving the above-described object, there is disclosed a method of providing a user interface for electrocardiogram analysis. The method may comprise: providing a first user interface that implements a visualized computing environment in which a first user can perform electrocardiogram analysis in order to generate basic reading information based on bio-data of an electrocardiogram reading target; and providing a second user interface that implements a visualized computing environment in which a second user can perform electrocardiogram analysis in order to generate final reading information based on the bio-data and the basic reading information generated via the first user interface. In this case, the first user interface and the second user interface may each include a first area adapted to display a first control graphic that switches a visual representation and state according to the result of electrocardiogram analysis. Furthermore, the first control graphic may be constructed for each type of disease or electrocardiogram feature that can be read from an electrocardiogram.

According to one embodiment of the present disclosure for achieving the above-described object, there is disclosed a computing device for providing a user interface for electrocardiogram analysis. The computing device may comprise: a processor including at least one core; memory including program codes executable by the processor; and an input/output unit configured to provide a user interface. In this case, the user interface may comprise a first area adapted to display a first control graphic that switches a visual representation and state according to the result of the electrocardiogram analysis performed based on bio-data of an electrocardiogram reading target. Furthermore, the first control graphic may be constructed for each type of disease or electrocardiogram feature that can be read from an electrocardiogram.

### Advantageous Effects

The present disclosure may provide the method of providing a user interface that allows a professional to easily perform electrocardiogram reading with simple operations and to intuitively become aware of information and review reading results.

### Description of Drawings

FIG. 1 is a block diagram of a computing device according to one embodiment of the present disclosure;
FIG. 2 is a conceptual diagram showing a screen that implements a user interface for electrocardiogram analysis according to one embodiment of the present disclosure;
FIG. 3 is a conceptual diagram showing a first control graphic according to one embodiment of the present disclosure;
FIG. 4 is a conceptual diagram showing a second control graphic according to one embodiment of the present disclosure;
FIG. 5 is a conceptual diagram showing an additional screen of a user interface according to one embodiment of the present disclosure;
FIG. 6 is a conceptual diagram showing a screen that implements a user interface for electrocardiogram analysis according to an alternative embodiment of the present disclosure;
FIG. 7 is a flowchart showing a method of providing a user interface for electrocardiogram analysis according to one embodiment of the present disclosure; and
FIG. 8 is a flowchart showing a method of providing a user interface for electrocardiogram analysis according to an alternative embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or **"unit"** may be defined in various manners within the range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The term "control graphic" used herein may be understood as a graphic object that executes a command to generate a visible result when a user interface is manipulated. In other words, the "control graphic" is a basic unit that constitutes part of a user interface, and may be understood as a user interface element that displays content to be provided to a user or enables a user to perform operation.

The foregoing descriptions of the terms are intended to help to understand the present disclosure. Accordingly, it should be noted that unless the above-described terms are explicitly described as limiting the content of the present disclosure, they are not used in the sense of limiting the technical spirit of the present disclosure.

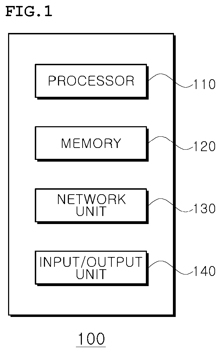

FIG. 1 is a block diagram of a computing device according to one embodiment of the present disclosure.

The computing device 100 according to the one embodiment of the present disclosure may be a hardware device or a part of a hardware device that performs the comprehensive processing and operation of data, or may be a software-based computing environment that is connected to a communication network. For example, the computing device 100 may be a server that is a principal agent performing an intensive data processing function and sharing resources, or may be a client that shares resources through interaction with a server or a specific terminal. Furthermore, the computing device 100 may be a cloud system in which pluralities of servers and clients comprehensively process data while interacting with each other. Furthermore, the computing device 100 may be a component of a medical robot. Since the above-described description is only one example related to the type of the computing device 100, the type of the computing device 100 may be configured in various manners within the range understandable to those skilled in the art based on the content of the present disclosure.

Referring to FIG. 1, the computing device 100 according to one embodiment of the present disclosure may include a processor 110, memory 120, a network unit 130, and an input/output unit 140. However, FIG. 1 shows only an example, and the computing device 100 may include other components for implementing a computing environment. Furthermore, only some of the components disclosed above may be included in the computing device 100.

The processor 110 according to one embodiment of the present disclosure may be understood as a constituent unit including hardware and/or software for performing computing operation. For example, the processor 110 may process commands generated as a result of user interaction via a user interface. Furthermore, the processor 110 may read a computer program and perform data processing for machine learning. The processor 110 may process operation processes such as the processing of input data for machine learning, the extraction of features for machine learning, and the computation of errors based on backpropagation. The processor 110 for performing data processing and operations may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within the range understandable to those skilled in the art based on the content of the present disclosure.

The processor 110 may generate a user interface that will be used by an electrocardiogram professional for the purpose of electrocardiogram reading. The user interface may include control graphics for outputting information necessary for performing electrocardiogram reading, receiving input according to the user operation, or implementing functions necessary for electrocardiogram reading. For example, the user interface may include a control graphic for outputting information included in the bio-data of an electrocardiogram reading target. The user interface may include a control graphic for visualizing the personal information of an electrocardiogram reading target, the waveform information of an electrocardiogram signal measured by the electrocardiogram reading target, etc. Furthermore, the user interface may include a control graphic for outputting the results of electrocardiogram reading performed based on output information or guiding a user to input them. The visual representation or state of each of the control graphics may be switched by a user operation.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within the range understandable to those skilled in the art based on the content of the present disclosure.

The memory 120 may structure, organize and manage data required for the processor 110 to perform operations, combinations of the data, and program codes executable by the processor 110. For example, the memory 120 may store the medical data received via the network unit 130 to be described later. The memory 120 may store the program code adapted to store rules for processing medical data, the program code adapted to operate a neural network model to receive medical data and perform training, the program code adapted to operate a neural network model to receive medical data and perform inference according to the purpose of use of the computing device 100, and the processed data generated as the program codes are executed.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive the data required for the processor 110 to perform operations through wired or wireless communication with any system, any client, or the like. Furthermore, the network unit 130 may transmit the data, generated through the operation of the processor 110, through wired or wireless communication with any system, any client, or the like. For example, the network unit 130 may receive bio-data through communication with a database within a hospital environment, a cloud server performing tasks such as the standardization of medical data, a client such as a smart watch, or a medical computing device. The network unit 130 may transmit output data of a neural network model, intermediate data and processed data obtained in the operation process of the processor 110, etc. through communication with the above-described database, server, client, or computing device.

The input/output unit 140 according to one embodiment of the present disclosure may be understood as a constituent unit including hardware and/or software for implementing a user interface. That is, the input/output unit 140 may visualize and output any type of data generated or determined by the processor 110 and any type of data received by the network unit 130. Furthermore, the input/output unit 140 may receive the user input that generates commands to be transmitted to any system or any client connected to the processor 110 or computing device 100 through wired or wireless communication. For example, the input/output unit 140 may include a display module that may output visualized information or implement a touch screen, such as a liquid crystal display (LCD), a thin film transistor-liquid crystal display (TFT LCD), an organic light-emitting diode (OLED), a flexible display, a 3D display, or the like. Furthermore, the input/output unit 140 may include an input module that can recognize a user's motion, voice, and/or other actions, such as a camera, a microphone, a keyboard, a mouse, and/or the like. Since the above-described modules are only examples, the modules included in the input/output unit 140 may be configured in various forms within the range understandable to those skilled in the art based on the content of the present disclosure in addition to the above-described examples.

The input/output unit 140 may implement a user interface, and may output the graphics generated via the processor 110 or may receive the user input generated by user operation and transmit it to the processor 110. For example, the input/output unit 140 may output a graphic related to the blood collection candidates generated via the processor 110, a graphic intended for the reception of the user input related to specific functions, a graphic intended for the provision of information, etc., in a specific area of the user interface. Furthermore, the input/output unit 140 may receive user input for the graphics output in a specific area of the user interface. In this case, the user input for a specific graphic may be understood as an input signal generated by a user operation of selecting the specific graphic via the input/output unit 140. Furthermore, the operation of selecting a specific graphic may refer to an action that can be performed by the user via the input/output unit 140, such as clicking, double-clicking, or hovering on the specific graphic. The input/output unit 140 may receive user input and transmit it to the processor 110, thereby enabling the operations of a blood collection device to be performed based on the user's control.

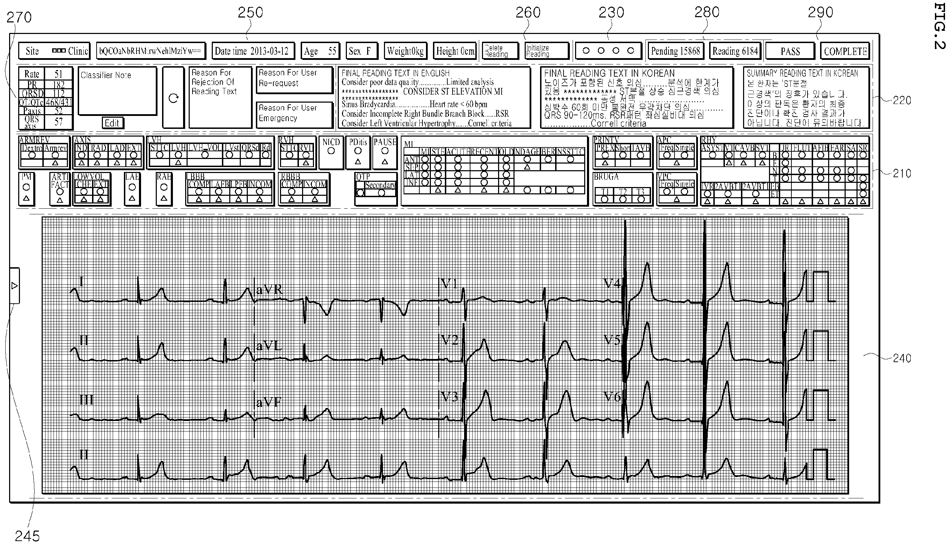

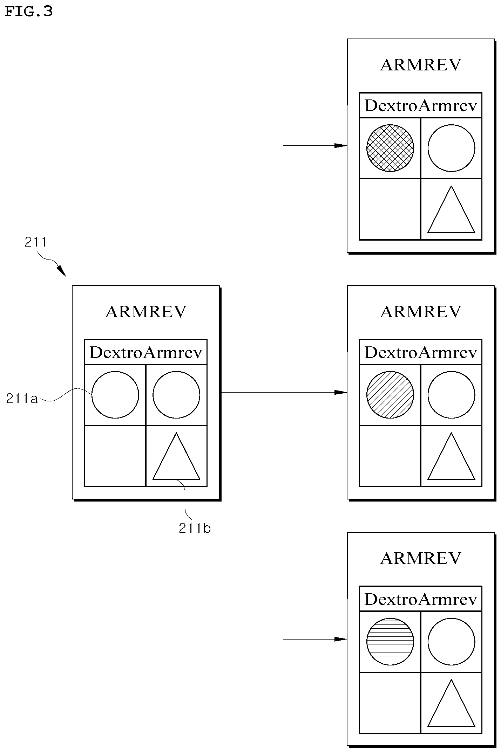

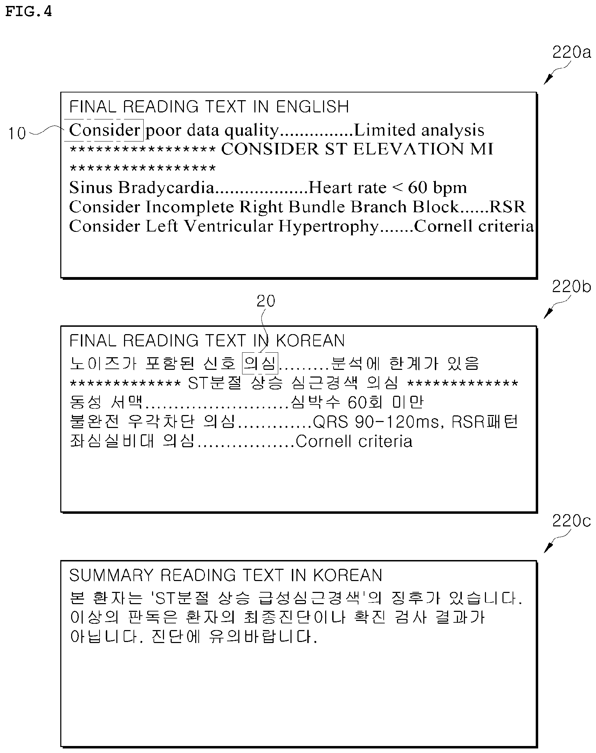

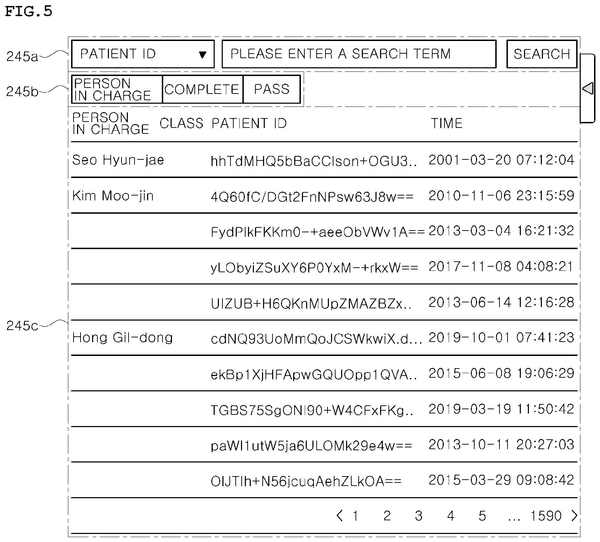

FIG. 2 is a conceptual diagram showing a screen that implements a user interface for electrocardiogram analysis according to one embodiment of the present disclosure. FIG. 3 is a conceptual diagram showing a first control graphic according to one embodiment of the present disclosure. FIG. 4 is a conceptual diagram showing a second control graphic according to one embodiment of the present disclosure. FIG. 5 is a conceptual diagram showing an additional screen of a user interface according to one embodiment of the present disclosure.

Referring to FIG. 2, a user interface according to one embodiment of the present disclosure may include a first area 210 that displays a first control graphic that switches a visual representation and state according to the result of the electrocardiogram analysis performed based on bio-data. The first control graphic displayed in the first area 210 is an element for displaying or inputting the result of electrocardiogram analysis, and may be constructed for each type of disease or electrocardiogram feature that can be read from an electrocardiogram. For example, the first control graphic may be a button-type graphic configured to display, input, or modify the result of electrocardiogram analysis for each type of disease that can be read from an electrocardiogram, such as left ventricular hypertrophy, cardiac arrest, myocardial infarction, or the like. Furthermore, the first control graphic may be a button-type graphic configured to display, input, or modify the result of electrocardiogram analysis for each electrocardiogram feature, such as the presence or absence of noise in an electrocardiogram signal, the direction of the axis of the heart that can be computed based on the electrocardiogram signal, or the like. When the computing operation of performing electrocardiogram analysis based on bio-data is completed, the first control graphic may dynamically switch a visual representation and state to display the result of the electrocardiogram analysis generated through the completion of the operation. Furthermore, when a user operation for inputting the result of electrocardiogram analysis is performed after an electrocardiogram has been analyzed based on bio-data, the first control graphic may dynamically switch a visual representation and state to display the result of the electrocardiogram analysis input through the user operation. That is, the first control graphic may be a button-type graphic that dynamically switches a visual representation and state in response to a command to input the result of electrocardiogram analysis. In this case, the command may be automatically generated when the operation of performing the electrocardiogram analysis is completed, or may be generated through a user operation for inputting the result of the electrocardiogram analysis.

More specifically, a first sub-control graphic may include at least one of the following: a first sub-control graphic that dynamically switches a visual representation and state according to an analysis result obtained by inputting electrocardiogram data included in bio-data to a pre-trained artificial intelligence model; a second sub-control graphic that dynamically switches a visual representation and state according to an analysis result obtained according to a logic predetermined for each type of disease or electrocardiogram feature that can be read from an electrocardiogram; and a third sub-control graphic that dynamically switches a visual representation and state based on a user operation for inputting an analysis result obtained by a user who analyzes an electrocardiogram.

The first sub-control graphic may be a button-type graphic that operates according to the output of a pre-trained artificial intelligence model. In this case, the artificial intelligence model may be a model pre-trained to extract features of an electrocardiogram waveform included in electrocardiogram data and individually output indicators for predicting a health condition based on the extracted features. The indicators for predicting a health condition may be understood to include analysis information related to a disease that affects health, such as whether a disease has occurred, the degree of aggravation of the disease, and the possibility of aggravation of the disease. The training of the artificial intelligence model may be performed based on supervised learning, or may be performed based on unsupervised learning or self-supervised learning. For example, when the artificial intelligence model receives electrocardiogram data included in bio-data and determines that myocardial infarction has occurred, the first sub-control graphic corresponding to the myocardial infarction determined to have occurred by the artificial intelligence model may be switched from an OFF state to an ON state. In this case, the first sub-control graphic may perform an operation such as a light emission or color change so that the user can recognize that the first sub-control graphic has been switched to an ON state. When the artificial intelligence model receives electrocardiogram data included in bio-data and determines that myocardial infarction has not occurred, the first sub-control graphic corresponding to the myocardial infarction determined not to have occurred by the artificial intelligence model may be maintained in an OFF state.

The second sub-control graphic may be a button-type graphic that operates according to a predetermined logic for each type of disease that can be read from an electrocardiogram. The predetermined logic may be a logic that derives a feature associated with at least one of the rhythm and shape of an electrocardiogram signal included in bio-data and determines whether a condition preset to determine the onset of a specific disease is satisfied by analyzing the derived feature. This logic may be automatically performed by a computing device (e.g., a server, or the like) that generates a user interface or a computing device (e.g., a client, or the like) that provides a user interface. For example, referring to FIG. 3, when a feature associated with at least one of the rhythm and shape of an electrocardiogram signal satisfies the onset condition of right-sided heart disease, a second sub-control graphic 211a in the form of a circle for right-sided heart disease may be switched from an OFF state to an ON state. In this case, the second sub-control graphic 211a in the form of a circle for right-sided heart disease may perform an operation such as a light emission or color change so that the user can recognize that it has been switched to an ON state, as shown in FIG. 3. When the feature associated with at least one of the rhythm and shape of the electrocardiogram signal does not satisfy the onset condition of right-sided heart disease, the second sub-control graphic 211a in the form of a circle for right-sided heart disease may be maintained in an OFF state. In this case, the onset condition may be preset based on the clinical experience or reading knowledge of an electrocardiogram reading professional.

The third control graphic may be a button-type graphic that operates according to a user operation for inputting an analysis result obtained by a user who analyzes an electrocardiogram. In this case, the user operation may be user input for a selection method. In this case, the selection method may include operation methods such as clicking, double-clicking, and right-clicking. For example, referring to FIG. 3, when the user determines that there is an electrode position abnormality, the third sub-control graphic 211b in the form of a triangle for the electrode position abnormality may be switched from an OFF state to an ON state through user input for a selection method. In this case, the third sub-control graphic 211b in the form of a triangle for the electrode position abnormality may perform an operation such as a light emission or color change so that the user can recognize that it has been switched to an ON state like the third sub-control graphic 211a for right-sided heart disease. When the user does not determine that there is an electrode position abnormality, the third sub-control graphic 211b in the shape of a triangle for the electrode position abnormality may be maintained in an OFF state because user input for a selection method is not received. When the user reverses the determination that there is an electrode position abnormality, the third sub-control graphic 211b in the shape of a triangle for the electrode position abnormality may be switched from the ON state to the OFF state through user input for a selection method. In this case, the user may determine whether there is an electrode position abnormality by analyzing an electrocardiogram signal based on his or her clinical experience or reading knowledge. Furthermore, the user may determine whether there is an electrode position abnormality by analyzing an electrocardiogram signal based on the preset guide rules provided by the user interface.

Meanwhile, the switching of the state of the first sub-control graphic or the second sub-control graphic may be performed by the reading of the computing device 100 rather than the user's reading. Accordingly, the first sub-control graphic or the second sub-control graphic may be switched from an ON state to an OFF state based on the user operation so that the user can verify and modify a reading result obtained by the computing device 100. For example, when the first sub-control graphic is switched from an OFF state to an ON state according to the reading result obtained using the artificial intelligence model but the user determines that the OFF state is appropriate, the user may perform an operation in the manner of selecting the state representation area of the first sub-control graphic. In this case, the method of selecting the state representation area may include operation methods such as clicking, double-clicking, and right-clicking. When the user input of selecting a state representation area is received, the first sub-control graphic may be switched from an ON state to an OFF state. The user may determine a reading result by easily reflecting his or her opinion in every reading result through such a simple operation.

The first sub-control graphic, the second sub-control graphic, or the third sub-control graphic may dynamically switch a color according to the severity readable based on the result of electrocardiogram analysis when being switched from an OFF state to an ON state. In this case, the color of the sub-control graphic may be determined based on the severity determined according to the type of disease that is signified by the sub-control graphic. For example, when anterior wall myocardial infarction is suspected among myocardial infarctions, the first sub-control graphic, the second sub-control graphic, or the third sub-control graphic for anterior wall myocardial infarction may be switched from an OFF state to an ON state while switching its color to black. When it is analyzed that acute anterior wall myocardial infarction is suspected, the first sub-control graphic, the second sub-control graphic, or the third sub-control graphic for acute anterior wall myocardial infarction may be switched from an OFF state to an ON state while switching a color to red. When the severity of the disease signified by the control graphic is low, the color of the control graphic may be displayed in black, whereas, when the severity of the disease signified by the control graphic is high, the color of the control graphic may be displayed in red. In other words, the disease signified by the control graphic displayed in red may refer to a medically severe disease that requires emergency treatment. In this case, the color may include black, green, yellow, and red in ascending order of the severity of the disease.

The first control graphic shown in the first area 210 may be reconstructed according to the number of leads of an electrocardiogram signal included in bio-data. When the number of leads of the electrocardiogram signal included in the bio-data is determined, the first control graphic may be reconstructed for each type of disease or electrocardiogram feature that can be read based on the number of leads of an electrocardiogram signal. For example, when the bio-data includes a 12-lead electrocardiogram, the first control graphic may be shown in the first area 210 according to the type of disease or electrocardiogram feature that can be read from the 12-lead electrocardiogram. When the bio-data includes a 6-lead electrocardiogram, the first control graphic may be displayed in the first area 210 according to the type of disease or electrocardiogram feature that can be read from the 6-lead electrocardiogram. In this manner, the first control graphic is not displayed in a fixed form in the first area 210, but may be dynamically reconstructed according to the number of leads of an electrocardiogram signal included in bio-data and may be displayed in the first area 210. The types of diseases or electrocardiogram features that can be read according to the number of leads may be arranged as shown in Table 1 below, but this is only one example for the understanding of the present disclosure, so that the present disclosure is not limited thereto.

**[Table 1]**

| Number of Leads | Readable Diseases |
|---|---|
| 12 Leads | atrial fibrillation, asystole, premature atrial contraction, premature ventricular contraction, sinus arrest, premature ventricular excitation syndrome, left axis deviation, right axis deviation, left atrial |
| | enlargement, right atrial enlargement, left bundle branch block, right bundle branch block, left ventricular hypertrophy, right ventricular hypertrophy, ST segment elevation myocardial infarction |
| 6 Leads | atrial fibrillation, asystole, premature atrial contraction, premature ventricular contraction, sinus arrest, premature ventricular excitation syndrome, left axis deviation, right axis deviation |
| 1 Lead | atrial fibrillation, asystole, premature atrial contraction, premature ventricular contraction |

Meanwhile, the forms of the sub-control graphics disclosed in FIGS. 2 and 3 and the four colors described above are only examples intended to help understanding of the present disclosure, and accordingly, the present disclosure is not limited to the above-described examples.

Referring to FIG. 2, a user interface according to one embodiment of the present disclosure may include a second area 220 that displays a second control graphic that displays a reading text generated by synthesizing the analysis results displayed via the first control graphic. The second control graphic may convert the per-disease analysis results, displayed via the first control graphic, into text and then display the text. In this case, the second control graphic may synthesize and display the per-disease analysis results, converted into text, in the form of a single reading text. For example, as shown in FIG. 3, the second control graphic may include a first reading text 220a in which English text is shown, a second reading text 220b in which Korean text is shown, and a third reading text 220c in which the analysis results are summarized and shown. The sentences shown in the first reading text 220a and the second reading text 220b may correspond to the per-disease analysis results displayed via the first control graphic. More specifically, when a first sub-control graphic or second sub-control graphic included in the first control graphic is in an ON state, the first reading text 220a and the second reading text 220b may show reading results corresponding to the analysis results, displayed via the first sub-control graphic or the second sub-control graphic in the ON state, in the form of text. When the sub-control graphics included in the first control graphic are in an ON state, the first reading text 220a and the second reading text 220b may also show tags that distinguish texts according to the types of sub-control graphics while displaying reading results corresponding to the analysis results, displayed via the sub-control graphics in an ON state, in the form of text. The first reading text 220a may show reading results corresponding to the analysis results, displayed via the third sub-control graphic, together with a first tag 10 such as "consider." The second reading text 220b may show reading results corresponding to the analysis results, displayed via the third sub-control graphic, together with a second tag 20 such as " 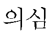 (suspect)." Furthermore, although not disclosed in FIG. 4, the first reading text 220a may show reading results corresponding to the analysis results, displayed via the first sub-control graphic, together with a third tag such as "probable." In this manner, the user may easily distinguish between the analysis results obtained by a computing operation and the analysis results obtained through his or her determination via the tags provided according to the type of sub-control graphic, and a third party may also intuitively distinguish the analysis results and additionally utilize them. Furthermore, the summary text shown in the third reading text 220c may correspond to the results of comprehensively evaluating the diseases that are predicted by synthesizing analysis results for individual diseases. Meanwhile, the first tag 10, second tag 20 and third tag described above are only examples for understanding of the content of the present disclosure, and accordingly, the tags of the present disclosure are not limited to the specific representations described above.

Referring to FIG. 2, a user interface according to one embodiment of the present disclosure may include a third area 230 that displays a third control graphic that displays the reading results, generated by synthesizing the analysis results displayed via the first control graphic, in color. When the analysis results are displayed in color according to the type of disease or electrocardiogram feature via the first control graphic, the severity of an electrocardiogram reading target read by synthesizing the analysis results may be represented by one of several colors via the third control graphic. For example, when as a result of synthesizing the analysis results displayed via the first control graphic, they are read as requiring an electrocardiogram test to be re-performed as necessary after professional review, the third control graphic may display a black color. When as a result of synthesizing the analysis results displayed via the first control graphic, they are read as not being associated with an emergency, the third control graphic may display a green color. When as a result of synthesizing the analysis results displayed via the first control graphic, they are read as requiring additional examination and treatment, the third control graphic may display a black color. When as a result of synthesizing the analysis results displayed via the first control graphic, they are read as being associated with an emergency, the third control graphic may display a red color. In this case, when there is at least one first control graphic that displays a red color corresponding to an emergency, the third control graphic may display a red color. When there is no first control graphic that displays a red color corresponding to an emergency and there is at least one first control graphic that displays a yellow color corresponding to a situation requiring additional examination and treatment, the third control graphic may display a yellow color. In this manner, the reading that determines the color displayed in the third control graphic may be performed based on the color indicative of the highest severity among the colors for the analysis results displayed via the first control graphic.

Referring to FIG. 2, a user interface according to one embodiment of the present disclosure may include a fourth area 240 that displays a fourth control graphic that displays an electrocardiogram signal included in bio-data according to the leads, a fifth area 245 that displays a fifth control graphic that displays a list of items for which electrocardiogram reading needs to be performed, a sixth area 250 that displays a sixth control graphic that displays the identification information included in bio-data, and a seventh area 260 that displays a seventh control graphic that receives the input intended for a user to adjust reading results. Furthermore, the user interface may include an eighth area 270 that displays an eighth control graphic that displays information necessary for electrocardiogram analysis and receives input for the recording of a user's opinion during an electrocardiogram analysis process, a ninth area 280 that displays a ninth control graphic that displays the state of performance of electrocardiogram analysis, and a tenth area 290 that displays a tenth control graphic that receives the input intended for a user to suspend or complete analysis.

The fourth control graphic displayed in the fourth area 240 may dynamically change the number of exposed signals according to the number of leads of an electrocardiogram signal included in bio-data. For example, when the number of leads of an electrocardiogram signal included in bio-data is 12, the fourth control graphic may display the signals of 12 leads. In this case, the fourth control graphic may perform operations such as the operation of reducing or enlarging an electrocardiogram signal, the operation of setting a point on an electrocardiogram signal, etc. based on the user input according to the user operation. Through these operations, the user may easily check the information required for electrocardiogram analysis by conveniently analyzing an electrocardiogram signal.

The fifth control graphic displayed in the fifth area 245 is a graphic that displays information about a target for which reading is to be performed, and may dynamically adjust the exposure of information according to the user operation. For example, in the configuration of a basic user interface shown in FIG. 2, when no separate user operation is performed on the fifth control graphic, the fifth control graphic may maintain an icon state without displaying any information. When a user operation is performed on the fifth control graphic, the fifth control graphic may list and display information about the target for reading as shown in FIG. 5. In this case, the fifth control graphic may include an entity 245a that displays an input window in which search can be performed based on the unique information of the target for electrocardiogram reading or time, a tab-type entity 245b that is intended to classify the state of the target for electrocardiogram reading as waiting, completed, or pass, and an entity 245c that displays information about the target for electrocardiogram reading according to the state classified in the tab-type entity.

The sixth control graphic displayed in the sixth area 250 may include an entity that displays information about a hospital in which an electrocardiogram signal was obtained, an entity that displays the unique information of a patient, from whom the electrocardiogram signal was obtained, as an unidentified random value, an entity that displays the date and time on and at which an electrocardiogram signal was obtained from an electrocardiogram reading target, and an entity that displays the age, gender, weight, and height of the electrocardiogram reading target. The information displayed in the entities included in the sixth control graphic may be derived through an operation that is automatically performed when a reading target is determined without a separate user input.

The seventh control graphic displayed in the seventh area 260 may include an entity that is used when the user deletes a reading result or restores a deleted result to a previous value, and an entity that is used when the user initializes a reading result. For example, when the user desires to delete specific information entered in the third sub-control graphic, the user may delete the information by manipulating the delete entity included in the seventh control graphic. In this case, the entity for deletion may be dynamically changed into the entity for restoration when a user operation is performed. Accordingly, when the user desires to restore previously deleted information after deletion, the user may delete the information by performing the operation of the entity for restoration that is generated as the entity for deletion is dynamically changed. In this case, the operation may be a user input for selecting an entity. Furthermore, when the user desires to restore all information entered in the third sub-control graphic to an initial state where no information was entered, the user may operate the entity for initialization included in the seventh control graphic to delete all entered information and restore it to an initial state.

The eighth control graphic displayed in the eighth area 270 may include an entity that lists features of an electrocardiogram signal. More specifically, the entity that lists features of an electrocardiogram signal may display the heart rate, PR interval, QRS interval, QT interval, P wave axis, and QRS axis that can be identified via the electrocardiogram signal. In this case, the information displayed in the corresponding entity may be obtained through an operation that is automatically performed when a reading target is determined without a separate user input. Furthermore, the eighth control graphic may include an entity that is intended to record the user's opinion, an entity that is used when the user modifies or reflects reading content, an entity that is intended to display other users' opinions, an entity that displays the reason for a request when re-reading is requested for a target for which reading has been completed, and an entity that displays the reason for a request when reading is requested for an emergency patient. The entity that displays other users' opinions may be used when a secondary reading user conveys to a primary reading user an opinion that reading needs to be corrected in the case where users performing electrocardiogram reading are divided into the primary reading user and the secondary reading user.

The ninth control graphic displayed in the ninth area 280 may include an entity that displays the number of electrocardiogram signals in a standby state that have not been read, and an entity that displays the number of electrocardiogram signals that have been read. The user may easily check the amount of work required to perform reading and reading performance via the ninth control graphic.

The tenth control graphic displayed in the tenth area 290 may include an entity that is used for the user to suspend electrocardiogram reading, and an entity that is used when a specific user transmits the reading result to another user. For example, when users performing electrocardiogram reading are divided into a primary reading user and a secondary reading user, the primary reading user may transmit a reading result to the secondary reading user by manipulating an entity included in the tenth control graphic.

Meanwhile, the positions and/or appearances of the areas included in the user interface according to the one embodiment of the present disclosure described with reference to FIG. 2 may be dynamically changed depending on the usage environment, and thus are not limited by the illustration of FIG. 2.

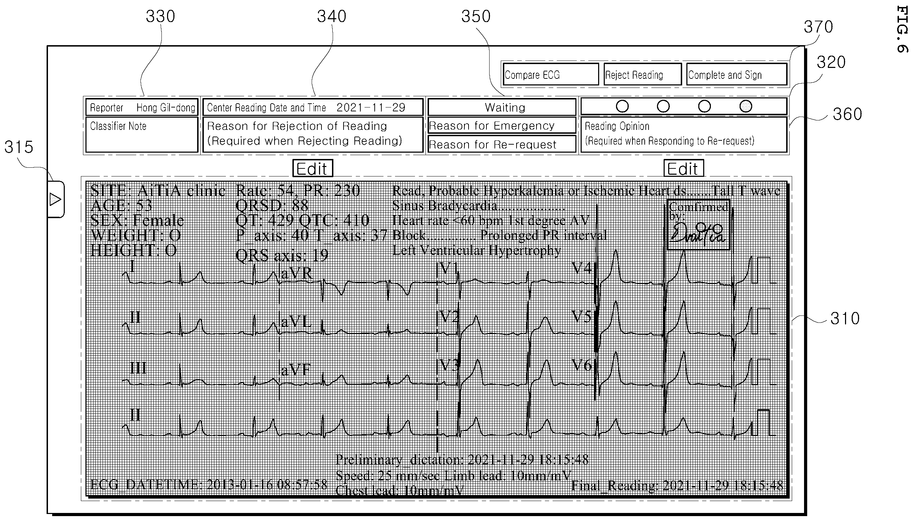

FIG. 6 is a conceptual diagram showing a screen that implements a user interface for electrocardiogram analysis according to an alternative embodiment of the present disclosure.

A user interface according to an alternative embodiment of the present disclosure may display control graphics for receiving and displaying the results of primary reading via the user interface of FIG. 2 or for receiving the input made by a user operation of performing secondary reading. More specifically, referring to FIG. 6, the user interface may include an eleventh area 310 that displays an eleventh control graphic that displays identification information about an electrocardiogram reading target, for which a first reading was performed, and an electrocardiogram signal, a twelfth area 315 that displays a twelfth control graphic that lists and displays information about the reading target, a thirteenth area 320 that displays a thirteenth control graphic that displays primary reading results in color, a fourteenth area 330 that displays a fourteenth control graphic that displays information about a user having performed the primary reading and items recorded during a reading process, a fifteenth area 340 that displays a fifteenth control graphic that displays information about the date and time on and at which the primary reading was performed and receives the reason for the rejection of reading from a user performing the secondary reading, a sixteenth area 350 that displays a sixteenth control graphic that displays an electrocardiogram request state and information corresponding to the request state, a seventeenth area 360 that displays a seventeenth control graphic that receives a final reading opinion from the user performing the secondary reading, and an eighteenth area 370 that displays an eighteenth control graphic that provides additional functions for the secondary reading.

The eleventh control graphic displayed in the eleventh area 310 corresponds to the form in which the fourth control graphic displayed in the fourth area 240 and the sixth control graphic displayed in the sixth area 250 are merged together and the twelfth control graphic displayed in the twelfth area 315 corresponds to the fifth control graphic displayed in the fifth area 245, so that detailed descriptions thereof will be omitted below. Furthermore, the thirteenth control graphic displayed in the thirteenth area 320 corresponds to the third control graphic displayed in the third area 230, so that a detailed description thereof will be omitted below.

The eighteenth control graphic displayed in the eighteenth area 370 may include an entity that is used to compare a plurality of electrocardiogram signals measured by the same target, an entity that is used for a user performing secondary reading to reject reading to a user performing primary reading, and an entity that is used to complete and store the secondary reading. For example, when the user operates the entity that is used to compare electrocardiogram signals, the entity may display all of the plurality of electrocardiogram signals measured by the same target in the same form as the eleventh control graphic. In this case, the other control graphics included in FIG. 6 may not be temporarily exposed, and only the plurality of electrocardiogram signals may be exposed.

Meanwhile, the locations and/or appearances of the areas included in the user interface according to the alternative embodiment of the present disclosure described with reference to FIG. 6 may be dynamically changed depending on the usage environment, and thus are not limited by the illustration of FIG. 6. Furthermore, the user interface according to the alternative embodiment of the present disclosure may be implemented not only as in FIG. 6, but also in the same form as in FIG. 2, depending on the purpose of use, and may also be implemented in the form in which all the elements shown in FIGS. 2 and 6 are merged together.

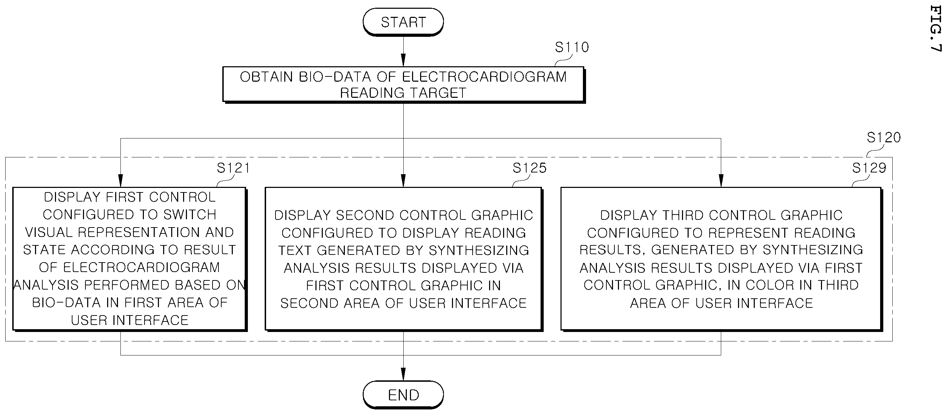

FIG. 7 is a flowchart showing a method of providing a user interface for electrocardiogram analysis according to one embodiment of the present disclosure.

Referring to FIG. 7, the computing device 100 according to one embodiment of the present disclosure may obtain bio-data of an electrocardiogram reading target in step S110. For example, the computing device 100 may receive the bio-data, measured by an electrocardiogram medical device, through communication with the electrocardiogram medical device. In this case, the bio-data may include the unique information of the electrocardiogram reading target and the electrocardiogram data measured from the electrocardiogram reading target. The electrocardiogram medical device may encrypt the bio-data and transmit it to the computing device 100, and the computing device 100 may encode the encrypted bio-data received from the electrocardiogram medical device and use it to generate an electrocardiogram analysis environment.

The computing device 100 may display a user interface for electrocardiogram analysis based on the bio-data, obtained via step S110, in step S120. For example, the computing device 100 may display a first control graphic configured to switch a visual representation and state according to the result of the electrocardiogram analysis performed based on the bio-data in the first area of the user interface in step S121. In this case, the first control graphic may be a button-type graphic that dynamically switches a visual representation and state in response to a command to input the result of the electrocardiogram analysis. The command to inputting the result of the electrocardiogram analysis may be automatically generated through an analysis operation performed by the computing device 100 based on the bio-data. In this case, the analysis operation performed by the computing device 100 may be performed based on an artificial intelligence model pre-trained to predict the onset of a specific disease, or may be performed according to a logic predetermined to determine whether the conditions for the onset of a specific disease are satisfied. Furthermore, the command to input the result of the electrocardiogram analysis may be generated through a user operation for inputting the result of the electrocardiogram analysis. In this case, the user operation may be the input performed in the manner of selecting a first control graphic constructed for each type of disease or electrocardiogram feature.

A user may intuitively become aware of the analysis results, obtained through the computing operation, at a glance via the first control graphic. Furthermore, the user may easily input analysis results for each disease and intuitively become aware of the input results at a glance via the first control graphic.

The computing device 100 may display a second control graphic configured to display a reading text generated by synthesizing the analysis results displayed via the first control graphic in the second area of the user interface in step S125. The computing device 100 may convert the analysis results, displayed via the first control graphic, into text. Furthermore, the computing device 100 may assign tags to the respective analysis results in order to distinguish the analysis results displayed via the first control graphic. More specifically, the computing device 100 may assign mutually distinguishable tags to the respective analysis results displayed via a first sub-control graphic that operates based on the operation results of the artificial intelligence model, the respective analysis results displayed via a second sub-control graphic that operates based on the operation results according to the predetermined logic, or the respective analysis results displayed via a third sub-control graphic that operates based on a user operation. The tags may be text elements preset for each type of sub-control graphic, and the representations of the tags may be determined by a user operation for each type of sub-control graphic. That is, the computing device 100 may assign tags to distinguish texts according to the type of sub-control graphic before or after the texts corresponding to the respective analysis results displayed via each of the sub-control graphics. The computing device 100 may generate a reading text by merging the texts converted through the above-described process into one. Furthermore, the computing device 100 may display the generated reading text via the second control graphic. Meanwhile, even when the analysis results are not displayed or input via the first control graphic, the basic frame of the second control graphic without content may be displayed in the second area.

The computing device 100 may display a third control graphic configured to represent the reading results, generated by synthesizing the analysis results displayed via the first control graphic, in color in the third area of the user interface in step S129. The computing device 100 may recognize a result having the highest severity among the analysis results displayed via the first control graphic. In this case, the severity may be an indicator predetermined according to the medical determination for each type of disease. For example, when it is suspected that right ventricular hypertrophy having a low level of emergency will occur, the first control graphic corresponding to right ventricular hypertrophy may display green indicative of a relatively low level of severity. When it is suspected that cardiac arrest having a high level of emergency will occur, the first control graphic corresponding to cardiac arrest may display red indicative of a considerably high level of severity. Furthermore, the computing device 100 may determine a color based on the recognized highest level of severity and display the determined color via the third control graphic. Meanwhile, even when no analysis results are displayed or input through the first control graphic, the basic frame of the third control graphic without content may be displayed in the third area.

The user may easily synthesize and organize the analysis results for each disease via the second control graphic, and may smoothly distinguish the analysis results via the tags. Furthermore, the user may intuitively become aware of the severities according to the reading results at a glance via the third control graphic.

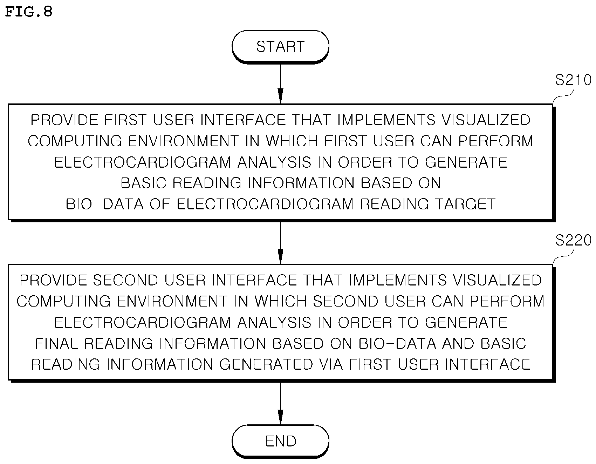

FIG. 8 is a flowchart showing a method of providing a user interface for electrocardiogram analysis according to an alternative embodiment of the present disclosure. The configuration of a first computing device and the configuration of a second computing device according to an alternative embodiment of the present disclosure may correspond to the configuration of the computing device 100 of FIG. 1 described above.

Referring to FIG. 8, according to an alternative embodiment of the present disclosure, the first computing device may provide a first user interface configured to implement a visualized computing environment in which a first user can perform electrocardiogram analysis in order to generate basic reading information based on bio-data of an electrocardiogram reading target in step S210. The first computing device may receive bio-data from an electrocardiogram medical device and output information necessary for electrocardiogram analysis via the first user interface. The first computing device may perform electrocardiogram analysis based on the bio-data and output analysis results via the first user interface. Furthermore, the first computing device may display control graphics configured to enable the first user to check information necessary for analysis and input analysis results via the first user interface. The first computing device may generate basic reading information based on the analysis results obtained through its own operation and the information input via the control graphics and display the basic reading information via the first user interface. Meanwhile, the first user interface may have a structure corresponding to that of the user interface of FIG. 2 described above.

The second computing device may provide a second user interface configured to implement a visualized computing environment in which a second user can perform electrocardiogram analysis in order to generate final reading information based on the bio-data and the basic reading information generated via the first user interface in step S220. The second computing device may obtain the bio-data and the basic reading information generated via the first user interface through communication with the first computing device, a cloud server for managing analysis results, or a database. The second computing device may output the bio-data and the basic reading information, generated via the first user interface, via the second user interface. The second computing device may display control graphics configured to enable the second user to check the information required for analysis and input analysis results or to modify or reject analysis results of the first user via the first user interface. The second computing device may display the final reading information, finalized by the second user, via the second user interface. Furthermore, the second computing device may store the final reading information, finalized by the second user, in various formats **(e.g.** XML format, CSV format, PDF format, etc.). Meanwhile, the second user interface may have a structure corresponding to that of the user interface of the FIG. 2 described above, or may have a structure corresponding to that of a user interface in the form in which the user interfaces of FIGS. 2 and 6 described above are merged together.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of providing a user interface for electrocardiogram analysis, the method comprising:
obtaining bio-data of an electrocardiogram reading target; and
displaying a first control graphic configured to switch a visual representation and state according to a result of electrocardiogram analysis performed based on the obtained bio-data in a first area of the user interface;
wherein the first control graphic is constructed for each type of disease or electrocardiogram feature that can be read from an electrocardiogram.

2. The method of claim 1, wherein:
the first control graphic is a button-type graphic configured to dynamically switch a visual representation and state in response to a command to input the result of the electrocardiogram analysis; and
the command is generated automatically when an operation of performing the electrocardiogram analysis is completed, or is generated through a user operation for inputting the result of the electrocardiogram analysis.

3. The method of claim 1, wherein the first control graphic comprises at least one of:
a first sub-control graphic configured to dynamically switch a visual representation and state according to an analysis result obtained by inputting electrocardiogram data, included in the obtained bio-data, to a pre-trained artificial intelligence model;
a second sub-control graphic configured to dynamically switch a visual representation and state according to an analysis result obtained according to a logic predetermined for each type of disease or electrocardiogram feature that can be read from the electrocardiogram; and
a third sub-control graphic configured to dynamically switch a visual representation and state based on a user operation for inputting an analysis result obtained by a user who analyzes the electrocardiogram.

4. The method of claim 3, wherein:
when the analysis result in which a disease that can be read from an electrocardiogram has occurred is obtained via the artificial intelligence model, the first sub-control graphic is switched from an OFF state to an ON state; and
when the analysis result in which a disease that can be read from an electrocardiogram has not occurred is obtained via the artificial intelligence model, the first sub-control graphic is maintained in an OFF state.

5. The method of claim 4, wherein, when being switched from an OFF state to an ON state, the first sub-control graphic dynamically switches a color according to severity that can be read based on the electrocardiogram analysis result obtained by the artificial intelligence model.

6. The method of claim 3, wherein, when a user input for a selection method is received, the first sub-control graphic is switched to an ON state or an OFF state.

7. The method of claim 3, wherein the predetermined logic:
derives a feature associated with at least one of a rhythm and shape of an electrocardiogram signal included in the obtained bio-data; and
determines whether a condition preset to determine an onset of a specific disease is satisfied by analyzing the derived feature.

8. The method of claim 7, wherein:
when it is determined that the feature satisfies the condition, the second sub-control graphic is switched from an OFF state to an ON state; and
when it is determined that the feature does not satisfy the condition, the second sub-control graphic is maintained in an OFF state.

9. The method of claim 8, wherein, when being switched from an OFF state to an ON state, the second sub-control graphic dynamically switches a color according to severity that can be read based on the analysis result obtained according to the predetermined logic.

10. The method of claim **3,** wherein, when a user input for a selection method is received, the second sub-control graphic is switched to an ON state or an OFF state.

11. The method of claim 3, wherein, when a user input for a selection method is received, the third sub-control graphic dynamically switches at least one of a color and a state.

12. The method of claim 1, further comprising displaying a second control graphic configured to display a reading text generated by synthesizing analysis results displayed via the first control graphic in a second area of the user interface.

13. The method of claim 12, wherein:
the reading text includes tags assigned to analysis results, respectively, in order to distinguish analysis results displayed via the first sub-control graphic in an ON state, analysis results displayed via the second sub-control graphic in an ON state, and analysis results displayed via the third sub-control graphic in an ON state;
the first sub-control graphic is included in the first control graphic, and operates based on analysis results obtained by inputting electrocardiogram data, included in the obtained bio-data, to a pre-trained artificial intelligence model;
the second sub-control graphic is included in the first control graphic, and operates based on analysis results obtained according to a logic predetermined for each type of disease or electrocardiogram feature that can be read from an electrocardiogram; and
the third sub-control graphic is included in the first control graphic, and operates based on a user operation for inputting analysis results obtained by a user who analyzes an electrocardiogram.

14. The method of claim 1, further comprising displaying a third control graphic configured to represent reading results, generated by synthesizing analysis results displayed via the first control graphic, in color in a third area of the user interface.

15. The method of claim 14, wherein the third control graphic dynamically switches a color according to severity read by synthesizing analysis results displayed via the first control graphic.

16. The method of claim 1, wherein, when a number of leads of an electrocardiogram signal included in the bio-data is determined, the first control graphic is reconstructed for each type of disease or electrocardiogram feature that can be read based on the number of leads of the electrocardiogram signal.

17. A method of providing a user interface for electrocardiogram analysis, the method comprising:
providing a first user interface that implements a visualized computing environment in which a first user can perform electrocardiogram analysis in order to generate basic reading information based on bio-data of an electrocardiogram reading target; and
providing a second user interface that implements a visualized computing environment in which a second user can perform electrocardiogram analysis in order to generate final reading information based on the bio-data and the basic reading information generated via the first user interface;
wherein the first user interface and the second user interface each include a first area adapted to display a first control graphic that switches a visual representation and state according to a result of electrocardiogram analysis; and
wherein the first control graphic is constructed for each type of disease or electrocardiogram feature that can be read from an electrocardiogram.

18. A computing device for providing a user interface for electrocardiogram analysis, the computing device comprising:
a processor including at least one core;
memory including program codes executable by the processor; and
an input/output unit configured to provide a user interface;
wherein the user interface includes a first area adapted to display a first control graphic that switches a visual representation and state according to a result of electrocardiogram analysis performed based on bio-data of an electrocardiogram reading target; and
wherein the first control graphic is constructed for each type of disease or electrocardiogram feature that can be read from an electrocardiogram.
